# EUROPEAN PATENT SPECIFICATION

(11) **EP 4 275 743 B1**
(45) Date of publication and mention of the grant of the patent: **02.04.2025**
(21) Application number: 22749775.7
(22) Date of filing: 03.02.2022
(51) Int. Cl.: A61N 5/10

(54) **BOLUS FORMING MATERIAL AND BOLUS USING SAME**
BOLUSBILDENDES MATERIAL UND BOLUS DAMIT
MATIÈRE DE FORMATION DE BOLUS ET BOLUS L'UTILISANT

(30) Priority: 05.02.2021 JP 2021017541
(43) Date of publication of application: 15.11.2023
(73) Proprietor: Hayakawa Rubber Co., Ltd., Fukuyama-shi, Hiroshima 721-8540 (JP); Kinki University, Higashi-Osaka-shi Osaka 577-8502 (JP)
(72) Inventor: MONZEN Hajime, Osaka-Sayama City, Osaka 5898511 (JP); TAMURA Mikoto, Osaka-Sayama City, Osaka 5898511 (JP); KADOWAKI Yoshito, Fukuyama-shi, Hiroshima 721-8540 (JP); NAKAMURA Masashi, Fukuyama-shi, Hiroshima 721-8540 (JP); SHIGITA Masafumi, Fukuyama-shi, Hiroshima 721-8540 (JP)
(74) Representative: Grünecker Patent- und Rechtsanwälte PartG mbB
(86) International application number: PCT/JP2022/004202
(87) International publication number: WO 2022/168904

(56) References cited:
- JP-A- 2018 076 478
- JP-A- 2018 076 478
- JP-A- H11 221 293
- JP-A- H11 221 294
- JP-A- H11 255 958
- US-A1- 2008 123 810
- US-A1- 2008 123 810

## Description

### Technical Field

The present invention relates to a bolus used in radiotherapy to adjust depth dose characteristics of radiation in which the dose is maximized at a location deeper than the skin.

### Background Art

Irradiation of the human body with radiations including X-rays, γ-rays, and electron beams is widely practiced for treating diseases such as cancer. In general, when a material is irradiated with radiation, the original amount of radiation decreases as the radiation goes deeper into the material. As a result, the dose decreases exponentially according to the depth of the material.

However, with the high-energy radiation, the direction of recoil electrons (scattered rays) is mainly in the radiation direction. Thus, the characteristics of high-energy radiation includes less lateral scattering, the dose reaching a maximum at a certain depth, this dose being higher than the surface dose, and afterwards the dose decreasing exponentially with further depth. Consequently, giving the characteristics of the scattered rays, the depth at which the dose is at a maximum must be adjusted. Otherwise, it is difficult to deliver a sufficient dose to the affected area near the skin surface, and harmful effects are exerted on normal tissues, apart from the affected area, by unnecessary radiation. For this reason, a tool called a bolus is placed between the radiation source and the skin to adjust the depth at which the dose is maximized.

The bolus is a tool that interposes a material having characteristics for radiation similar to those of human body between the radiation emitting device and the human body, and the bolus adjusts the dose to be maximized at the affected area. However, poor adhesion between the human body and the bolus results in a decrease in the dose delivered to the affected area. Further, in radiotherapy where the same affected area is treated with multiple bouts of irradiation, the bolus application position is less reproducible over multiple treatment sessions.

The bolus is required to have the following characteristics.
(1) Be a material equivalent to human tissue
(2) Be homogeneous
(3) Have excellent plasticity, appropriate elasticity, and good shape adaptability and adhesion to living body
(4) Have no toxicity
(5) Exhibit no energy changes and the like
(6) Be of uniform thickness
(7) Not be contaminated by air exposure

Furthermore, transparency is preferred because the degree of adhesion to the human body can be visually recognized therewith. Note that the material equivalent to the human tissue described herein refers to any material that has the same radiation absorption and scattering characteristics as the human body. For example, the material is acceptable if a profile of the percentage depth dose, when irradiated with radiation, is the same as that of an equivalent to the human body or water. Furthermore, it is also important that the material can be placed on the patient's skin surface during CT imaging at the time of treatment planning, that the application position is highly reproducible, and that the material does not cause artifacts.

Patent Literature 1 discloses a bolus formed by pouring a bolus-forming liquid material containing water, a mineral, and a polymerizable monomer into a mold and then curing. Herein, the mold is produced using a three-dimensional printer on the basis of the shape data of the patient's skin surface.

The bolus of Patent Literature 1 is intended to provide the bolus having the shape along the patient's body surface to be irradiated with radiation and also having a radiation transmittance distribution corresponding to the patient's affected area.

Furthermore, Patent Literature 2 discloses, as a bolus, a hydrous gel of a natural organic polymer compound. This hydrous gel is produced by adding one or two or more natural organic polymers (carrageenan, locust bean gum, glucomannan, starch, curdlan, guar gum, agar, cassia gum, dextran, amylose, gelatin, pectin, xanthan gum, tara gum, and gellan gum) as a gelling formulation to water at a concentration that causes gelation or higher. The amount of the gelling formulation relative to water is preferably 10% or less, more preferably 2 to 5%.

Patent Literature 2 intends to provide a bolus that is disposable, can be produced at a lower cost than the conventional bolus, and has excellent cleanliness and processability.

### Citation List

### Patent Literature

PTL 1: Japanese Patent Application Laid-Open No. 2020-58905
PTL 2: Japanese Patent Application Laid-Open No. Hei. 11-255958

### Summary of Invention

### Technical Problem

It is preferable that the bolus be inserted between the radiation source of radiation and the affected area to shift the profile of the dose (change the dose) in the depth direction and also that there be no gap between the bolus and the human body surface. In this respect, the bolus provided in Patent Literature 1 is suitable because it is produced by pouring the bolus material into a mold produced using a three-dimensional printer on the basis of shape data on the patient's skin surface and curing, aligning perfectly with the shape (uneven) of the skin surface. However, it is necessary to produce the mold by measuring the patient's skin surface shape. In an actual treatment setting, adjusting the bolus based on the situation at hand is thus difficult.
Furthermore, there is a problem in that the homogeneity of the bolus itself cannot be maintained due to a change in the material filling rate (variation in thickness) when the bolus material is poured into the mold produced using the three-dimensional printer.

On the other hand, the bolus provided by Patent Literature 2 is easy to use in that it is simple and disposable. However, radiation therapy usually treats the same affected area with multiple bouts of irradiation. Thus, the same bolus needs to be applied to the affected area each time. That is, it is required that the bolus not change its shape for a certain period of time, or that it return to its original shape even if the shape changes. The bolus according to Patent Literature 2 is not stable in terms of shape retention. Furthermore, it has poor adhesion to an affected area with an uneven surface.

### Solution To Problem

The present invention has been conceived in view of the above-mentioned problems and provides a bolus forming material whose shape hardly changes over time during treatment and can be relatively easily matched to the shape (uneven) of a human body surface, and a bolus formed by using this bolus forming material.

More specifically, the bolus forming material according to the present invention is characterized in that:
the bolus forming material has a 30°C rubber hardness A of 20 degrees or more, and a 70°C rubber hardness E of 60 degrees or less and 10 degrees or more; and
the bolus forming material shifts a peak of the percentage depth dose for electron beams and X-rays in a direction of a beam source by 0.8 to 1.2 times the thickness thereof.

Furthermore, a bolus according to the present invention is characterized by being formed by the above-mentioned bolus forming material which is formed into a plate shape with a thickness of 0.1 mm or more and 50 mm or less. Advantageous Effects of Invention

The bolus according to the present invention has a 30°C rubber hardness A of 20 degrees or more and a 70°C rubber hardness E of 60 degrees or less and 10 degrees or more, making it possible to provide a bolus that easily deforms at 70°C but hardly deforms at 30°C. Furthermore, since the dose characteristics in the depth direction are mostly the same as those of the human body, the peak position of the dose can be adjusted by adjusting the thickness. Furthermore, in a radiotherapy treatment setting, a bolus that matches the shape of the affected area can be formed simply by heating the bolus. Furthermore, since the deformed shape is maintained at normal temperature, the bolus molded to fit the affected area can be used repeatedly and hygienically by wrapping it with a plastic wrap or the like.

### Brief Description of Drawings

[Fig.1] Fig. 1 is a graph showing a temperature-dependent change of a rubber hardness A measured by changing a chemical composition of a bolus forming material.
[Fig. 2] Fig. 2 is a graph showing a temperature-dependent change of a rubber hardness E measured by changing the chemical composition of the bolus forming material.
[Fig. 3] Fig. 3 is a diagram for explaining measurement systems for a percentage depth dose.
[Fig. 4] Fig. 4 is graphs showing measurement results of the percentage depth doses when a thickness of the bolus forming material is set to 5 mm and 10 mm.

### Description Of Embodiments

A bolus forming material and a bolus according to the present invention will be described below with reference to an example. Note that the following description shows one embodiment and one example of the present invention, and the present invention is not limited to the following description. The following description can be modified without departing from the gist of the present invention. Further, in the present invention, "A to B" means "A or more and B or less".

The bolus according to the present invention is formed of a bolus forming material having viscoelastic temperature characteristics, wherein the bolus forming material is hard or elastic enough not to be plastically deformed in shape at body temperature, and can be easily plastically deformed when heated hot within a temperature range where the bolus forming material can be touched with a hand. Furthermore, this bolus forming material has mostly the same dose characteristics in the depth direction compared with the human body.

More specifically, the bolus forming material is a rubber composition that contains a rubber material as a main material, has a 30°C rubber hardness A of 20 degrees or more, and has a 70°C rubber hardness E of 60 degrees or less.

Furthermore, the peak of the percentage depth dose for electron beams and X-rays needs to be shifted in the direction of a beam source by 0.8 to 1.2 times the thickness. The thickness described herein is the length of the bolus forming material in the direction of the radiation axis when the bolus forming material is placed on a line connecting the radiation axis from the radioactive beam source and the irradiation target (usually a patient's affected area). In normal circumstances, it may be the thickness of the bolus forming material formed into a plate shape.

There are several methods for measuring rubber hardness. However, the standard is "JIS (Japanese Industrial Standards) K6253 Rubber, vulcanized or thermoplastic - Determination of hardness". The rubber hardness conforming to this measurement method is generally used. As the measurement principle, a needle of a predetermined size is attached at the end of a spring with a certain elastic modulus, and the measurement is performed by examining how far this needle can be pushed down.

Examples of the type of rubber hardness include Type A (hereinafter referred to as (type A)), Type D (hereinafter referred to as (type D)), and Type E (hereinafter referred to as (type E)). Since the hardness of a rubber has a wide range, the hardness cannot be evaluated with just one spring. Thus, the measurement range is covered by multiple springs with different elastic moduli.

As a rough guide, an object that has a hardness of 20 degrees or less with a type A measuring instrument is ideally measured by a type E measuring instrument, and an object that has a hardness of 90 degrees or more with the type A measuring instrument is ideally measured by a type D measuring instrument.

Furthermore, according to a type A value, a hardness of 10 is as hard as human skin, a hardness of 40 is as hard as an eraser, a hardness of 65 is as hard as a car tire, and a hardness of 90 is as hard as a golf ball.

Rubber hardness is described with notation in the order of a measurement temperature, a measurement type, and a measured value. For example, the above phrase "30°C rubber hardness A of 20 degrees or more" indicates that the hardness of the measured object set at 30°C, measured with a type A measuring instrument, is 20 degrees or more. Furthermore, the phrase "70°C rubber hardness E of 40 degrees or less" indicates that the hardness of the measured object warmed to 70°C, measured with a type E measuring instrument, is 40 degrees or less.

If the bolus forming material has a 30°C rubber hardness A of 20 degrees or more, it can maintain a shape deformed to match the shape of the affected area while being elastic and not undergoing plastic deformation. Furthermore, if the bolus forming material has a thickness of about ten-odd millimeters, it is minimally compressed and deformed in the thickness direction. As a result, the shape can be maintained during irradiation with the radiation, and the scattering of the dose in the depth direction can be reliably adjusted.

On the other hand, if the bolus forming material has a 70°C rubber hardness E of 60 degrees or less, the bolus forming material heated to 70°C can be touched without difficulty by using rubber gloves, which is almost the same as using bare hands, making it possible to form the bolus into a shape according to the shape of the affected area.

Furthermore, the bolus forming material of the present invention has a dose profile in the depth direction (referred to as "dose characteristics in the depth direction") that is mostly the same as that of the human body. More specifically, when the electron beams and X-rays used in radiotherapy are emitted, the peak of the percentage depth dose shifts in the direction of the beam source by 0.8 to 1.2 times the thickness of the bolus. For example, by using the bolus with a thickness of 5 mm, the peak of the percentage depth dose shifts by 4 mm to 6 mm in the direction of the radiation source.

The percentage depth dose is a percentage of the dose at a given depth, the maximum dose on the central axis of radiation in water being 100%. A specific method for measuring the percentage depth dose will be described in detail in an example described below.

The bolus according to the present invention is completed by forming the bolus forming material into a plate shape of a predetermined thickness. Specifically, the plate shape has a thickness of 0.1 mm or more and 50 mm or less. The size of the plate is not particularly limited, but a size of about 250 mm x 250 mm is highly convenient for transportation and handling during use. Furthermore, an object obtained by molding the plate-shaped bolus into the shape of the affected area is also called a bolus. Molding into a plate shape can be performed by a method such as flattening the bolus forming material with a rolling pin or passing the bolus forming material through rollers set with a certain gap.

A method of using the bolus according to the present invention is as follows. First, a bolus having been molded in a plate shape with a desired thickness is cut into a size that sufficiently covers the affected area to be irradiated with radiation. The bolus (bolus forming material) of the present invention can be easily cut with scissors at normal temperature.

Next, the cut bolus is heated to about 70°C. This heating can plastically deform the bolus. Anticipating the shape of the affected area, the heated bolus is shaped to roughly the same shape. Then, when the temperature reaches a temperature range where the bolus can be directly brought into contact with the skin surface, such as when the temperature reaches about 50°C for example, the bolus is directly applied to the affected area and pressed from above so that it conforms to the shape of the affected area. By pressing, the shape of the bolus is adjusted while the bolus is closely adhered to the shape of the skin surface. At this time, it is possible to adjust the shape in a simple manner by applying hot air or the like to partially heat the bolus to make fine corrections.

Then, the bolus that has been made into the shape of the skin surface is cooled. The cooling may be performed by leaving the bolus as it is, or by cooling the bolus with cold air or the like. When the temperature returns to normal, the bolus of the present invention, deformed to match the shape of the affected area, maintains the deformed shape, making it possible to obtain a bolus that is closely adhered to the skin at the affected area. At this time, in order to improve adhesion, it is preferable that the bolus be tightly wrapped from the outside with a bandage, a plastic wrap, or the like. Furthermore, at this time, the bolus, after being deformed, has a certain degree of elasticity, and thus the bolus can be closely adhered to the skin when it is applied again to the skin to be the affected area. Furthermore, by utilizing the heat deformation characteristics of the bolus, even if the shape of the affected area changes for some reason (e.g., the patient loses weight) during the treatment period, the bolus can be partially deformed again to match the affected area.

Note that a bolus forming material with a nonuniform thickness or a bolus thicker than the desired thickness is heated, and when it becomes soft, it is adjusted to have a predetermined thickness, applied to the patient's skin surface, and molded into the skin surface shape. Further, a procedure of cutting off unnecessary parts with scissors after cooling may be included. With this method, the bolus thickness can be adjusted in practical settings.

Furthermore, it is more preferable that the bolus itself be transparent at the time of bolus formation, as transparency would make it possible to form the bolus while examining the affected area. The phrase "at the time of bolus formation" described herein refers to a temperature range in which the bolus can be directly brought into contact with the skin surface. Specifically, the range is 30°C to 55°C, and more preferably 40°C to 50°C. That is, the bolus according to the present invention has transparency when heated. Furthermore, the degree of transparency may be such that the marking of the treatment site can be confirmed when the bolus is brought into close contact with the skin. Optically, the bolus may be semi-transparent.

In the object of the present invention, a configuration of the bolus forming material is not particularly limited as long as the above-mentioned rubber hardness and dose characteristics in the depth direction are satisfied. However, as confirmed by the applicants, a rubber composition composed of a main material and an auxiliary material is preferable. Details will be given below.

### <Main material>

This is the main material of the bolus forming material and its content is 60 to 70% by mass of the whole. For changing the rubber hardness by temperature, a general method is to use a main material that has a high (hard) rubber hardness even at a certain high temperature and adjust the rubber hardness on the high temperature side with an auxiliary material. More specifically, the rubber hardness at the high temperature side and that at the low temperature side are adjusted by mixing the auxiliary material with temperature characteristics of rubber hardness that are different from those of the main material.

A rubber material called an elastomer can be used as the main material. Examples of the main material include a natural rubber and a synthetic rubber such as a styrene-butadiene rubber, a butadiene rubber, an ethylene-propylene rubber, a butyl rubber, a urethane rubber, a silicone rubber, and a fluorine rubber, and a thermoplastic elastomer such as styrene-based, olefin/alkene-based, vinyl chloride-based, urethane-based, and amide-based thermoplastic elastomers. At least one type of these materials can be suitably used. Moreover, multiple types thereof may be used as a mixture. As the main material, an ethylene-propylene rubber or the like may be particularly preferably used.

### <Auxiliary material>

In order for the bolus forming material to have suitable temperature characteristics of rubber hardness and to have appropriate elongation and strength, the main material alone is not sufficient, and the auxiliary material is used. As the auxiliary material, a temperature-sensitive material, a reinforcing agent, a softening agent, and the like are used.

### (Temperature sensitive material)

The temperature sensitive material is used to adjust the temperature characteristics of the main material. For example, if the rubber material as the main material is too soft at 36°C, which is the temperature of the human skin, using a thermoplastic resin that is still hard at that temperature can increase the rubber hardness at around 36°C.

Furthermore, the thermoplastic characteristics can be exhibited by adjusting the chemical composition of the rubber material. A material that, together with the main material, provides the temperature characteristics of the bolus forming material as a whole is called a temperature sensitive material. The temperature sensitive material is a material that has the temperature characteristics of the rubber hardness different from those of the main material.

### (Softening agent)

The temperature range in which the bolus is used is from normal temperature 20°C to about 70°C. The softening agent may be used as a material that can adjust the overall viscosity of the bolus forming material in this temperature range. In general, an oil can be suitably used as the softening agent. When the softening agent is used, it is contained in an amount that does not cause blooming during storage.

### (Reinforcing agent)

The reinforcing agent is added to maintain a structure of the bolus forming material. For example, a filler component can be suitably used. The filler component in use is a fine particle, and can be inorganic, organic, or both. The main materials that incorporate an appropriate amount of the filler component can increase the bonding strength between them. Since the filler component has no temperature characteristics, it supports the structural strength of the main material in the temperature range in which it is intended to be used (between about 20°C and about 70°C).

Note that the bolus according to the present invention is required to have the same radiation characteristics as the human body. Thus, if too much high-density filler is added, the radiation characteristics of the bolus deviates too far from the radiation characteristics of the human body, which is not preferable. Thus, the above-mentioned materials are mixed such that the bolus is formed so as to have substantially the same radiation characteristics as the human body.

More specifically, it is desirable that the specific gravity fall within a range of 0.9 to 1.2 g/cm³. If it is smaller than this, the thickness of the bolus needs to be increased in order to achieve the desired peak shift amount, making handling of the bolus difficult. Furthermore, if it exceeds this range, the bolus may deviate too far from the radiation characteristics of the human body.

However, the specific gravity is only a rough indication, and the bolus according to the present invention is only required that it can shift the peak of the percentage depth dose for electron beams and X-rays in the direction of the beam source by 0.8 to 1.2 times the thickness. For a 10 mm-thick bolus, this corresponds to the ability to shift the peak of the percentage depth dose by 8 mm to 12 mm to the beam source side.

### Examples

### <Example 1> Temperature characteristics

As the main material, ethylene-propylene rubbers (EPT4021, EPT4045, EPTX-4010M: all manufactured by Mitsui Chemicals Inc.) were used. As the temperature sensitive material, a styrene-butadiene rubber (Asaprene 6500P: manufactured by Asahi Kasei Corp.) was used. The ratio of the main material to the temperature sensitive material was about 4:1.

As the other auxiliary materials, two types of auxiliary materials, including Diana Process Oil PW-380 (manufactured by Idemitsu Kosan Co., Ltd.) as a softening agent (processed oil), and silica (Nip Seal AQ: manufactured by Tosoh Silica Corp.) and talc (Mistron Vapor: manufactured by Imerys Specialties Japan Co., Ltd.) as a reinforcing agent, were used.

The chemical composition of each sample is shown below.

Sample 1 (hereinafter referred to as "SP1") was prepared with the following chemical composition.

### Main material: ethylene-propylene rubber

| | |
|---|---|
| EPT4021 | 60 parts by weight (38.7% by mass) |
| EPT4045 | 0 parts by weight (0.0% by mass) |
| EPTX-4010M | 20 parts by weight (12.9% by mass) |
| Total main material | 80 parts by weight (51.6% by mass) |
| Temperature sensitive material: | styrene-butadiene rubber 20 parts by weight (12.9% by mass) |
| Auxiliary material: softening agent: | processed oil 20 parts by weight (12.9% by mass) |
| Reinforcing agent: | Silica 35 parts by weight (22.6% by mass) |
| Reinforcing agent: | Talc 0 parts by weight (0% by mass) |

Sample 2 (hereinafter referred to as "SP2") was prepared with the following chemical composition.

### Main material: ethylene-propylene rubber

| | |
|---|---|
| EPT4021 | 80 parts by weight (51.6% by mass) |
| EPT4045 | 0 parts by weight (0.0% by mass) |
| EPTX-4010M | 0 parts by weight (0.0% by mass) |
| Total main | material 80 parts by weight (51.6% by mass) |
| Temperature sensitive material: | styrene-butadiene rubber 20 parts by weight (12.9% by mass) |
| Auxiliary material: | softening agent: processed oil 20 parts by weight (12.9% by mass) |
| Reinforcing agent: | Silica 35 parts by weight (22.6% by mass) |
| Reinforcing agent: | Talc 0 parts by weight (0% by mass) |

Sample 3 (hereinafter referred to as "SP3") was prepared with the following chemical composition.

### Main material: ethylene-propylene rubber

| | |
|---|---|
| EPT4021 | 70 parts by weight (45.2% by mass) |
| EPT4045 | 0 parts by weight (0.0% by mass) |
| EPTX-4010M | 0 parts by weight (0.0% by mass) |
| Total main material | 70 parts by weight (45.2% by mass) |
| Temperature sensitive material: | styrene-butadiene rubber 30 parts by weight (19.4% by mass) |
| Auxiliary material: softening agent: | processed oil 20 parts by weight (12.9% by mass) |
| Reinforcing agent: | Silica 35 parts by weight (22.6% by mass) |
| Reinforcing agent: | Talc 0 parts by weight (0% by mass) |

Sample 4 (hereinafter referred to as "SP4") was prepared with the following chemical composition.

### Main material: ethylene-propylene rubber

| | |
|---|---|
| EPT4021 | 0 parts by weight (0.0% by mass) |
| EPT4045 | 60 parts by weight (38.7% by mass) |
| EPTX-4010M | 20 parts by weight (12.9% by mass) |
| Total main material | 80 parts by weight (51.6% by mass) |
| Temperature sensitive material: | styrene-butadiene rubber 20 parts by weight (12.9% by mass) |
| Auxiliary material: softening agent: | processed oil 20 parts by weight (12.9% by mass) |
| Reinforcing agent: | Silica 35 parts by weight (22.6% by mass) |
| Reinforcing agent: | Talc 0 parts by weight (0% by mass) |

Sample 5 (hereinafter referred to as "SP5") was prepared with the following chemical composition.

### Main material: ethylene-propylene rubber

| | |
|---|---|
| EPT4021 | 60 parts by weight (40.0% by mass) |
| EPT4045 | 0 parts by weight (0.0% by mass) |
| EPTX-4010M | 30 parts by weight (20.0% by mass) |
| Total main material | 90 parts by weight (60.0% by mass) |
| Temperature sensitive material: | styrene-butadiene rubber 10 parts by weight (6.7% by mass) |
| Auxiliary material: softening agent: | processed oil 20 parts by weight (13.3% by mass) |
| Reinforcing agent: | Silica 0 parts by weight (0.0% by mass) |
| Reinforcing agent: | Talc 30 parts by weight (20.0% by mass) |

The respective chemical compositions are collectively shown in Table 1 and Table 2.

**[Table 1]**

| | | | Name of Sample | | | | | |
|---|---|---|---|---|---|---|---|---|
| Item | Material | Product Number | SP1 | | SP2 | | SP3 | |
| | | | Part by Weight | % by Mass | Part by Weight | % by Mass | Part by Weight | % by Mass |
| Main Material | EPDM | EPT 4021 | 60 | 38.7 | 80 | 51.6 | 70 | 45.2 |
| | | EPT 4045 | 0 | 0.0 | 0 | 0.0 | 0 | 0.0 |
| | | EPT X-4010M | 20 | 12.9 | 0 | 0.0 | 0 | 0.0 |
| Total Amount of Main Material | | | 80 | 51.6 | 80 | 51.6 | 70 | 45.2 |
| Temperature Sensitive Material | SBR | Asaprene 6500P | 20 | 12.9 | 20 | 12.9 | 30 | 19.4 |
| Softening Agent | Processed Oil | Diana Process Oil PW-380 | 20 | 12.9 | 20 | 12.9 | 20 | 12.9 |
| Reinforcing Agent | Silica | Nip Seal AQ | 35 | 22.6 | 35 | 22.6 | 35 | 22.6 |
| | Talc | Mistron Vapor | 0 | 0.0 | 0 | 0.0 | 0 | 0.0 |
| | | total | 155 | 100.0 | 155 | 100.0 | 155 | 100.0 |
| Specific Gravity (g/cm³) | | | 0.99 | | 0.99 | | 0.99 | |

| | | | | | | | | |
|---|---|---|---|---|---|---|---|---|
| EPDM: Ethylene-Propylene Rubber SBR: Styrene-Butadiene Rubber | | | | | | | | |

**[Table 2]**

| | | | Name of Sample | | | |
|---|---|---|---|---|---|---|
| Item | Material | Product Number | SP4 | | SP5 | |
| | | | Part by Weight | % by Mass | Part by Weight | % by Mass |
| Main Material | EPDM | EPT 4021 | 0 | 0.0 | 60 | 40.0 |
| | | EPT 4045 | 60 | 38.7 | 0 | 0.0 |
| | | EPT X-4010M | 20 | 12.9 | 30 | 20.0 |
| Total Amount of Main Material | | | 80 | 51.6 | 90 | 60.0 |
| Temperature Sensitive Material | SBR | Asaprene 6500P | 20 | 12.9 | 10 | 6.7 |
| Softening Agent | Processed Oil | Diana Process Oil PW-380 | 20 | 12.9 | 20 | 13.3 |
| Reinforcing Agent | Silica | Nip Seal AQ | 35 | 22.6 | 0 | 0.0 |
| | Talc | Mistron Vapor | 0 | 0.0 | 30 | 20.0 |
| | | total | 155 | 100.0 | 150 | 100.0 |
| Specific Gravity (g/cm³) | | | 0.99 | | 1.00 | |

| | | | | | | |
|---|---|---|---|---|---|---|
| EPDM: Ethylene-Propylene Rubber SBR: Styrene-Butadiene Rubber | | | | | | |

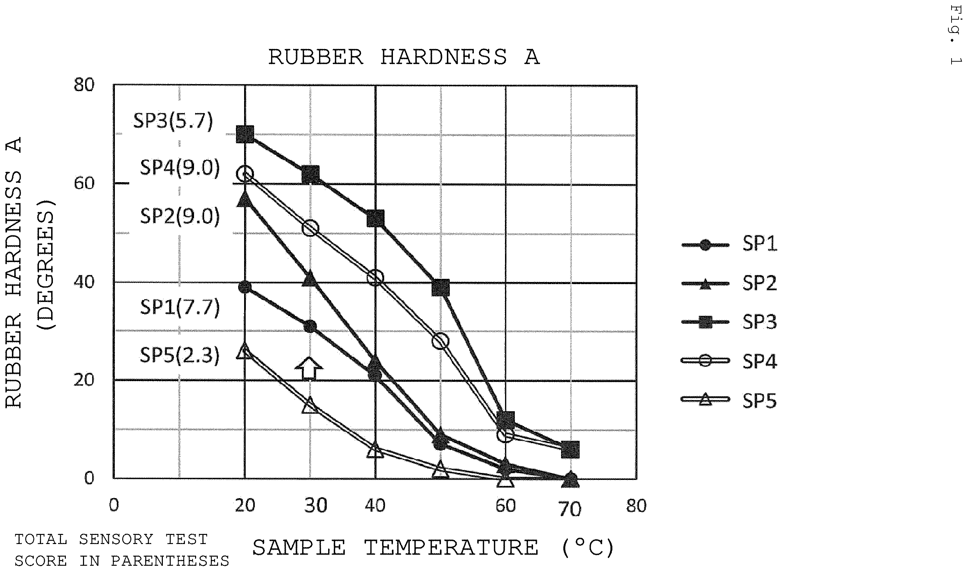

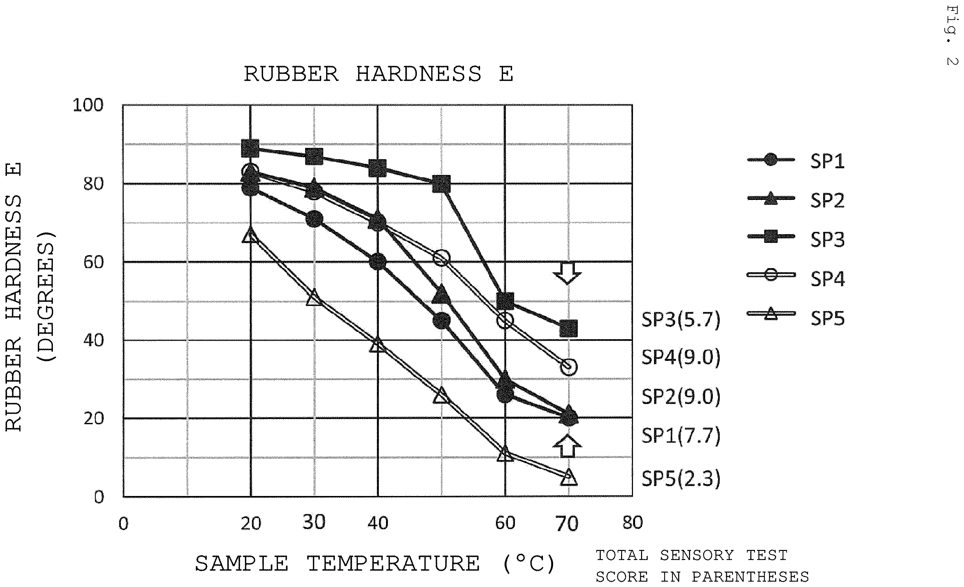

These samples were then molded into plate shapes of 50 mm × 50 mm × 10 mm, and placed in a constant temperature bath at a predetermined temperature for 30 minutes. Then, the samples that reached the temperature of the constant temperature bath were taken out, and the rubber hardness was measured with type A and type E rubber hardness meters. The results are shown in Fig. 1 and Fig. 2. Fig. 1 shows the rubber hardness measured by the type A rubber hardness meter, and Fig. 2 shows the rubber hardness by the type E rubber hardness meter.

In both Fig. 1 and Fig. 2, the horizontal axis represents the sample temperature (°C) and the vertical axis represents the rubber hardness (degree). Fig. 1 and Fig. 2 show that the rubber hardness tends to decrease from the low temperature side to the high temperature side, and the order of samples, in terms of rubber hardness, does not change between 20°C and 70°C during measurement. In order of decreasing rubber hardness, the order was found to be Sample 3, Sample 4, Sample 2, Sample 1, and Sample 5.

The Type A rubber hardness meter exhibited high sensitivity when the rubber hardness was 20 degrees or more and showed clear differences between respective samples. However, when the rubber hardness was 20 degrees or less, the sensitivity decreased, and thus clear differences between the respective samples could not be shown. On the other hand, the Type E rubber hardness meter exhibited decreased sensitivity when the rubber hardness was 60 degrees or more, and exhibited high sensitivity when the rubber hardness was less than 60 degrees, and thus clear differences between the respective samples could be shown.

Each sample having such temperature characteristics of rubber hardness was evaluated in a setting where the sample was actually handled by a person. A human sensory test will be described. Each sample was given a random number and molded into a plate shape of 100 mm × 100 mm × 10 mm. Examiners were three radiological technologists from a radiology department. Each sample was left in a constant temperature bath at 70°C or 36°C for 30 minutes. The sample was kneaded by hands, and evaluated on a 5-point scale focusing on moldability at 70°C and shape retention at 36°C. The evaluation was performed within 2 minutes. Evaluation criteria are as follows.
5 Usable in clinical practice without problems, and easy to use.
4 Usable in a clinical setting without problems
3 Usable in a clinical setting
2 Usable in a clinical setting with some problems
1 Not usable in a clinical setting

For each sample, average scores of the moldability at 70°C and the shape retention at 36°C by the three examiners were calculated, and the average scores were summed up to obtain a total score. Table 3 and Table 4 show the evaluation results and the rubber hardness at 30°C and 70°C for each sample. Furthermore, the total sensory test score for each sample was also shown in Fig. 1 and Fig. 2.

**[Table 3]**

| Sensory Test and Rubber Hardness | | | | | | | | | |
|---|---|---|---|---|---|---|---|---|---|
| Name of Sample | SP1 | | | SP2 | | | SP3 | | |
| | Hardness | | Sensory Test Score | Hardness | | Sensory Test Score | Hardness | | Sensory Test Score |
| | TypeA | Type E | | TypeA | Type E | | TypeA | TypeE | |
| 30°C (Sensory test was performed at 36°C.) | 31 | | 3.7 | 41 | | 4.7 | 62 | | 3.3 |
| 70°C | | 20 | 4.0 | | 21 | 4.3 | | 43 | 2.3 |
| Total Sensory Test Score | | | 7.7 | | | 9.0 | | | 5.7 |

**[Table 4]**

| Sensory Test and Rubber Hardness | | | | | | |
|---|---|---|---|---|---|---|
| Name of Sample | SP4 | | | SP5 | | |
| | Hardness | | Sensory Test Score | Hardness | | Sensory Test Score |
| | TypeA | Type E | | TypeA | TypeE | |
| 30°C (Sensory test was performed at 36°C.) | 51 | | 4.3 | 15 | | 1.0 |
| 70°C | | 33 | 4.7 | | 5 | 1.3 |
| Total Sensory Test Score | | | 9.0 | | | 2.3 |

Reference is made to Table 3 and Table 4, and Fig. 1 and Fig. 2. First, referring to Table 3 and Table 4, Sample 1, and Sample 2, and Sample 4 all had an average score of 3 points (usable in a clinical setting) or more at temperature of 36°C and 70°C. In particular, Samples 2 and 4 had, in the sensory test, 4 points (usable in a clinical setting without problems) or more at both temperatures, producing a very favorable result.

Sample 3 was in the 2-point range (usable in a clinical setting with some problems) at 70°C and in the 3-point range (usable in a clinical setting) at 36°C.

On the other hand, Sample 5 was in the 1-point range (not usable in a clinical setting) at both 36°C and 70°C. According to Fig. 1, this indicates that if the 30°C rubber hardness A is 20 degrees or more, preferably 25 degrees or more, and most preferably 30 degrees or more, the sample can be closely adhered to the patient's skin without experiencing plastic deformation while on the human skin.

Next, referring to Fig. 2, if the rubber hardness is too high on the high temperature side, deforming the sample becomes difficult, which is not preferable. Furthermore, from the result of SP5, the rubber hardness being too low makes handling not preferable. As the upper limit, the 70°C rubber hardness E is 60 degrees or less, preferably 50 degrees or less, and most preferably 45 degrees or less. Furthermore, as the lower limit, the 70°C rubber hardness E is 10 degrees or more, preferably 15 degrees or more, and most preferably 20 degrees or more. Within these ranges, the sample can be freely deformed in a clinical setting.

As described above, if the 30°C rubber hardness A is 20 degrees or more and the 70°C rubber hardness E is 60 degrees or less and 10 degrees or more, it becomes possible to provide a bolus forming material that can be suitably used in a clinical setting.

Furthermore, when Sample 1, Sample 2, Sample 3, and Sample 4 were heated, the boluses became semi-transparent. This showed that the samples were sufficiently transparent for confirming the marking at the treatment site through the bolus. Thus, Sample 1, Sample 2, Sample 3, and Sample 4 had favorable workability for molding the bolus.

### <Example 2> Dose characteristics in depth direction

The bolus according to the present invention can be favorable in terms of mechanical handling by setting the temperature characteristics within a predetermined range. On the other hand, as a fundamental role of the bolus, the radiological characteristics need to be similar to those of the human body.

Thus, in order to evaluate whether the bolus of the present invention has the same characteristics of radiation absorption or scattering as the actual tissues, the percentage depth dose for electron beams and X-rays was measured. The percentage depth dose is a percentage of the dose at a given depth, the maximum dose on the central axis of radiation in water being 100%.

### (Measuring method)

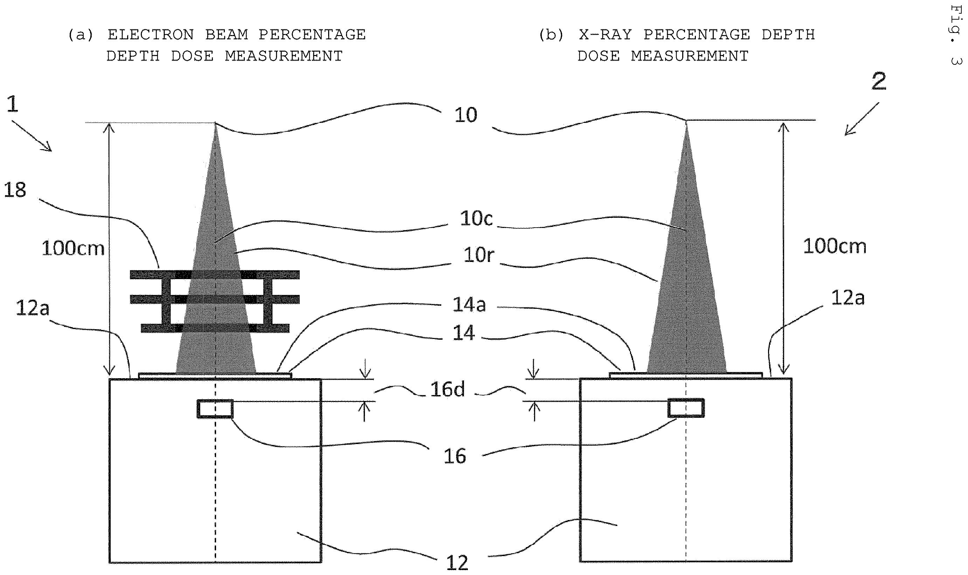

Fig. 3(a) and Fig. 3(b) show configurations of measurement systems. Fig. 3(a) is for electron beam percentage depth dose measurement 1, and Fig. 3(b) is for X-ray percentage depth dose measurement 2. Common to both measurement systems, an absorbed dose measurement phantom 12 (hereinafter also simply referred to as "phantom 12") was placed below a beam source 10, and a bolus 14 molded to a predetermined thickness was placed on a surface 12a of the phantom 12.

As the phantom 12, Tough Water Phantom (Kyoto Kagaku Co., Ltd.) was used. The phantom 12 is made from a material that interacts with radiation, in terms of absorption and scattering for example, in an equivalent manner to the way water and human tissues do. Furthermore, this material has a similar density to water and human tissues.

The distance between the beam source 10 and the phantom surface 12a was set to 100 cm. The irradiation field was 10 × 10 cm². Furthermore, in the electron beam percentage depth dose measurement 1, an applicator 18 was placed between the beam source 10 and the bolus 14. The applicator 18 was placed to prevent side scattering of the electron beams in the air.

The percentage depth dose was acquired by using a measuring instrument 16 (Roos type plane-parallel ionization chamber (PTW Freiburg GmbH) and electrometer RAMTEC SMART (Toyo Medic Co., Ltd.)). Both were positioned on a radiation axis 10c of a radiation 10r from the beam source 10 and at a predetermined depth 16d from the phantom surface 12a.

The electron beam energy was 4 to 12 MeV, and the X-ray energy was 4, 6 and 10 MV. A linear accelerator Synergy (Elekta K. K.) was used to generate electron beams and X-rays.

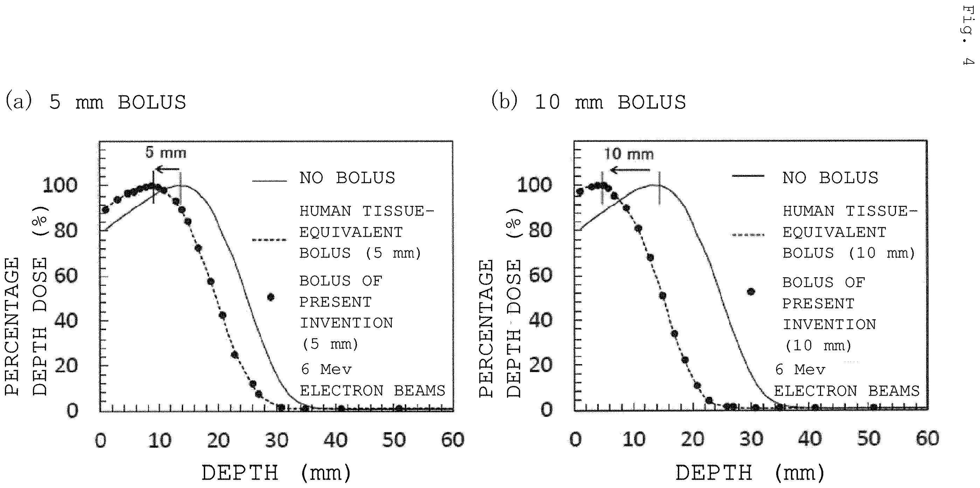

Note that the human tissue-equivalent bolus (CIVCO Radiotherapy) was placed on the phantom 12 in the same manner, and the percentage depth dose was acquired under the same conditions and compared with that of the bolus of the present invention (SP1 was used). Both thicknesses were 5 mm and 10 mm. Fig. 4 shows, as an example, the result when the electron beam energy was 6 MeV.

Fig. 4(a) shows the result in the case where the thickness of the bolus 14 was 5 mm, and (b) shows the result in the case where the thickness was 10 mm. In both graphs, the horizontal axis represents the depth from the bolus surface 14a (when there was no bolus, the depth (mm) from the surface 12a of the phantom 12), and the vertical axis represents the percentage depth dose (%).

Furthermore, in both graphs, the solid black line represents no bolus, and the dashed black line represents the human tissue equivalent bolus. The bolus (SP1) according to the present invention is represented by black circles.

Referring to Fig. 4, there are two cases in terms of bolus thickness, 5 mm (Fig. 4(a)) and 10 mm (Fig. 4(b)). In both cases, the percentage depth doses of the bolus according to the present invention and the human tissue equivalent bolus are almost the same, and the bolus 14 according to the example showed the same radiation characteristics as the existing human tissue equivalent bolus. The same tendency was observed with the electron beam energy of 4 MeV, 9 MeV, and 12 MeV, and X-ray energy of 4, 6, and 10 MV.

Regarding the effect of the bolus, the peak of the percentage depth dose was shifted by 5 mm toward the beam source direction by the bolus with 5 mm thickness, and by 10 mm by the bolus with thickness of 10 mm. Including the case where the electron beam intensity was changed, with the bolus according to the present invention, the peak was shifted by 4 mm to 6 mm when the bolus thickness was 5 mm, and shifted by 8 mm to 10 mm when the bolus thickness was 10 mm. Thus, with the bolus according to the present invention, the peak of the percentage depth dose for electron beams and X-rays can be shifted in the direction of the beam source by 0.8 to 1.2 times the thickness of the bolus.

When radiation is used for treatment, the energy to be used is usually fixed. A desired shift amount can be obtained by researching beforehand the shift amount for the energy of the radiation. That is, the shift amount of the percentage depth dose toward the beam source side can be controlled by the thickness of the bolus according to the present invention.

### Industrial Applicability

The bolus formed from the bolus forming material according to the present invention can be suitably used to shift the percentage depth dose toward the beam source side at the time of radiotherapy.

### Reference Signs List

1 electron beam percentage depth dose measurement
2 X-ray percentage depth dose measurement
10 beam source
10r radiation
10c radiation axis
12 absorbed dose measurement phantom
12a surface (of phantom 12)
14 bolus
14a bolus surface
16 measuring instrument
16d depth (from phantom surface 12a)
18 applicator

## Claims

1. A bolus forming material wherein:
the bolus forming material has a 30°C rubber hardness A of 20 degrees or more, and a 70°C rubber hardness E of 60 degrees or less and 10 degrees or more; and
the bolus forming material can shift a peak of a percentage depth dose for electron beams and X-rays in a direction of a beam source by 0.8 to 1.2 times a thickness thereof.

2. The bolus forming material according to claim 1, comprising a rubber composition, wherein
the rubber composition includes
an ethylene-propylene rubber as a main material, and
a temperature sensitive material.

3. The bolus forming material according to claim 1 or 2, further comprising a reinforcing agent.

4. A bolus comprising the bolus forming material according to any of claims 1 to 3, the bolus forming material being formed into a plate shape with a thickness of 0.1 mm or more and 50 mm or less.

5. The bolus forming material according to any one of claims 1 to 4, having a transparency that allows one to confirm a marking at a treatment site through the bolus forming material during heating.

## Patentansprüche

1. Bolusbildendes Material, wobei:
das bolusbildende Material eine 30°C-Gummihärte A von 20 Grad oder mehr und eine 70°C-Gummihärte E von 60 Grad oder weniger und 10 Grad oder mehr aufweist, und
das bolusbildende Material die Spitze der relativen Tiefendosis für Elektronenstrahlen und Röntgenstrahlen in der Richtung einer Strahlquelle um das 0,8 bis 1,2-fache der Dicke verschieben kann.

2. Bolusbildendes Material nach Anspruch 1, das eine Gummizusammensetzung umfasst,
wobei die die Gummizusammensetzung umfasst:
ein Ethylen-Propylen-Gummi als ein Hauptmaterial, und
ein temperaturempfindliches Material.

3. Bolusbildendes Material nach Anspruch 1 oder 2, das weiterhin ein Verstärkungsmittel umfasst.

4. Bolus, der das bolusbildende Material gemäß einem der Ansprüche 1 bis 3, umfasst, wobei das bolusbildende Material zu einer Plattenform mit einer Dicke von 0,1 mm oder mehr und 50 mm oder weniger geformt ist.

5. Bolusbildendes Material nach einem der Ansprüche 1 bis 4, das eine Transparenz aufweist, mit der eine Markierung an einer Behandlungsstelle durch das bolusbildende Material hindurch während des Erwärmens bestätigt werden kann.

## Revendications

1. Matériau de formation de bolus dans lequel :
le matériau de formation de bolus a une dureté de caoutchouc A à 30°C de 20 degrés ou plus, et une dureté de caoutchouc E à 70°C de 60 degrés ou moins et de 10 degrés ou plus ; et
le matériau de formation de bolus peut déplacer le pic **d'un** pourcentage de dose en profondeur pour les faisceaux d'électrons et les rayons X dans la direction d'une source de faisceau de 0,8 à 1,2 fois son épaisseur.

2. Matériau de formation de bolus selon la revendication 1, comprenant une composition de caoutchouc, dans lequel
la composition de caoutchouc inclut
un caoutchouc éthylène-propylène comme matériau principal, et
un matériau sensible à la température.

3. Matériau de formation de bolus selon la revendication 1 ou 2, comprenant en outre un agent de renforcement.

4. Bolus comprenant le matériau de formation de bolus selon l'une quelconque des revendications 1 à 3, le matériau de formation de bolus étant formé en forme de plaque avec une épaisseur de 0,1 mm ou plus et de 50 mm ou moins.

5. Matériau de formation de bolus selon l'une quelconque des revendications 1 à 4, ayant une transparence qui permet de confirmer un marquage sur un site de traitement à travers le matériau de formation de bolus pendant le chauffage.
